# EUROPEAN PATENT APPLICATION

(11) **EP 4 624 574 A1**
(43) Date of publication of application: **01.10.2025**
(21) Application number: 23893954.0
(22) Date of filing: 23.11.2023
(51) Int. Cl.: C12N 15/11, C12N 15/63, A61K 48/00, A61K 38/00

(54) **UTR MOLECULE FOR INCREASING PROTEIN EXPRESSION LEVEL**

(30) Priority: 24.11.2022 WO PCT/CN2022/134038
(71) Applicant: Suzhou Abogen Biosciences Co., Ltd., Suzhou, Jiangsu 215123 (CN)
(72) Inventor: QIN, Qianshan, Suzhou, Jiangsu 215123 (CN); YUAN, Lei, Suzhou, Jiangsu 215123 (CN); ZHOU, Nishui, Suzhou, Jiangsu 215123 (CN); XU, Jun, Suzhou, Jiangsu 215123 (CN); GAO, Peng, Suzhou, Jiangsu 215123 (CN)
(74) Representative: Epping - Hermann - Fischer
(86) International application number: PCT/CN2023/133522
(87) International publication number: WO 2024/109866

(57) **Abstract**

The present disclosure relates to a UTR molecule and use thereof. Specifically, the present disclosure relates to an mRNA molecule comprising 5' UTR and 3' UTR. The UTR molecule can significantly increase mRNA expression level.

## Description

The present application claims priority to PCT Application No. PCT/CN2022/134038 filed on November 24, 2022, which is incorporated herein by reference in its entirety.

### TECHNICAL FIELD

The present disclosure relates to a UTR molecule and use thereof. Specifically, the present disclosure relates to an mRNA molecule comprising 5' UTR and 3' UTR. The UTR molecule can significantly increase the protein expression level of the mRNA molecule.

### BACKGROUND

As a gene therapy, mRNA vaccine is a very promising drug. However, mRNA is unstable and easy to be degraded by ubiquitous RNAase. In vivo, RNA degradation can help to modulate RNA half-life and fine-tune the regulation of eukaryotic gene expression (Friedel CC, Dölken L, Ruzsics Z, Koszinowski UH, Zimmer R. Nucleic Acids Res. 2009; 37(17): e115-e115. doi:10.1093/NAR/GKP542). Stable RNA is necessary for the expression level of RNA drugs. There are many methods to regulate the stability of nucleic acid molecules, including regulating the GC content of nucleic acids (WO2002098443A2), adding UTR (untranslated region) sequences, 5' cap (Galloway A, Cowling VH. Biochim Biophys Acta - Gene Regul Mech. 2019; 1862(3): 270-279. doi:10.1016/j.bbagrm.2018.09.011), 3' polyA tail (Tudek A, Lloret-Llinares M, Heick Jensen T. Philos Trans R Soc B Biol Sci. 2018; 373(1762). doi:10.1098/rstb.2018.0169).

UTR is a key factor for translation efficiency (Jackson RJ, Hellen CUT, Pestova T V. Nat Rev Mol Cell Biol 2010 112. 2010; 11(2):113-127. doi:10.1038/nrm2838). The 3' UTR of α-globin mRNA is known to play an important role in the stability of α-globin mRNA (Rodgers ND, Wang Z, Kiledjian M. RNA. 2002; 8(12): 1526. doi:10.1017/s1355838202029035). The 3' UTR of α-globin mRNA is involved in the formation of a specific nucleoprotein-complex (α-complex) and is associated with the in vitro stability of mRNA (Wang Z, Day N, Trifillis P, Kiledjian M. Mol Cell Biol. 1999; 19(7): 4552. doi:10.1128/MCB.19.7.4552). Based on the experimental data of 280,000 randomized 5' UTRs, Moderna constructed a neural network and designed a universal 5' UTR that increases the expression level (Sample PJ, Wang B, Reid DW, et al. Nat Biotechnol. 2019; 37(7): 803-809. doi:10.1038/s41587-019-0164-5; US10881730B2). BioNtech screened out 5UTRs and 3UTRs that can increase expression through fragment libraries (US 2022/0273820 A1; Orlandini von Niessen AG, Poleganov MA, Rechner C, et al. Mol Ther. 2019; 27(4): 824-836. doi:10.1016/j.ymthe.2018.12.011).

In spite of the aforementioned progress, there is still an urgent need for UTR molecular combinations that can stabilize mRNA molecules and increase their protein expression level.

### SUMMARY OF THE INVENTION

The inventors of the present application unexpectedly discovered that the specific 5' UTR and 3' UTR combination described herein can significantly increase the protein expression level of mRNA molecules, and the mRNA can thus be advantageously used in gene therapy, gene vaccination, protein replacement therapy, antisense therapy or treatment by interfering RNA.

**In one aspect,** the present disclosure provides an mRNA molecule comprising 5' UTR and 3' UTR, wherein the 5' UTR is selected from the following (1)-(5):
(1) 5' UTR comprising an RNA sequence corresponding to any of the nucleic acid sequences set forth in SEQ ID NOs: 1-22, a homologue, fragment or variant thereof, wherein the homologue, fragment or variant has the same or better function of improving translation efficiency compared with 5' UTR comprising an RNA sequence corresponding to any of the nucleic acid sequences set forth in SEQ ID NOs: 1-22; preferably, the nucleic acid sequence of the homologue has at least 70%, 80%, 85%, 90%, 91%, 92%, 93%, 94%, 95%, 96%, 97%, 98% or 99% identity with an RNA sequence corresponding to any of the nucleic acid sequences set forth in SEQ ID NOs: 1-22;
(2) 5' UTR consisting of an RNA sequence corresponding to any of the nucleic acid sequences set forth in SEQ ID NOs: 1-22;
(3) 5' UTR consisting of an RNA sequence corresponding to any of the nucleic acid sequences set forth in SEQ ID NOs: 4, 3, 12, 17 or 19-22;
(4) 5' UTR obtained from two or more identical 5' UTRs in the aforementioned (1)-(3) linked in tandem; or
(5) 5' UTR obtained from two or more different 5' UTRs in the aforementioned (1)-(3) linked in tandem,
and wherein the 3' UTR is selected from the following (1)-(5):
(1) 3' UTR comprising an RNA sequence corresponding to any of the nucleic acid sequences set forth in SEQ ID NOs: 23-36, a homologue, fragment or variant thereof, wherein the homologue, fragment or variant has the same or better function of improving translation efficiency compared with 3' UTR comprising an RNA sequence corresponding to any of the nucleic acid sequences set forth in SEQ ID NOs: 23-36; preferably, the nucleic acid sequence of the homologue has at least 70%, 80%, 85%, 90%, 91%, 92%, 93%, 94%, 95%, 96%, 97%, 98% or 99% identity with an RNA sequence corresponding to any of the nucleic acid sequences set forth in SEQ ID NOs: 23-36;
(2) 3' UTR consisting of an RNA sequence corresponding to any of the nucleic acid sequences set forth in SEQ ID NOs: 23-36;
(3) 3' UTR consisting of an RNA sequence corresponding to any of the nucleic acid sequences set forth in SEQ ID NOs: 23, 24, 28 or 29;
(4) 3' UTR obtained from two or more identical 3' UTRs in the aforementioned (1)-(3) linked in tandem; or
(5) 3' UTR obtained from two or more different 3' UTRs in the aforementioned (1)-(3) linked in tandem;
and wherein the 5' UTR and 3' UTR combination does not include RNA sequences corresponding to the nucleic acid sequences set forth below: SEQ ID NO: 22 and SEQ ID NO: 23, SEQ ID NO: 20 and SEQ ID NO: 24, and SEQ ID NO: 21 and SEQ ID NO: 25.

In some embodiments, the 5' UTR is selected from an RNA sequence corresponding to the nucleic acid sequence set forth in SEQ ID NO: 4, 3, 12, 17, 19, 20, 21 or 22.

In some embodiments, the 3' UTR is selected from an RNA sequence corresponding to the nucleic acid sequence set forth in SEQ ID NO: 23, 24, 25, 28 or 29.

In some embodiments, the 5' UTR is selected from an RNA sequence corresponding to the nucleic acid sequence set forth in SEQ ID NO: 19 or 22, and the 3' UTR is selected from an RNA sequence corresponding to the nucleic acid sequence set forth in SEQ ID NO: 23, 24, 25, 28 or 29.

In some embodiments, the 5' UTR is selected from an RNA sequence corresponding to the nucleic acid sequence set forth in SEQ ID NO: 4, 3 or 12, and the 3' UTR is selected from an RNA sequence corresponding to the nucleic acid sequence set forth in SEQ ID NO: 23, 24, 28 or 29.

In some embodiments, the 5' UTR is an RNA sequence corresponding to the nucleic acid sequence set forth in SEQ ID NO: 17, and the 3' UTR is selected from an RNA sequence corresponding to the nucleic acid sequence set forth in SEQ ID NO: 23, 24 or 28.

In some embodiments, the 5' UTR is selected from an RNA sequence corresponding to the nucleic acid sequence set forth in SEQ ID NO: 20 or 21, and the 3' UTR is selected from an RNA sequence corresponding to the nucleic acid sequence set forth in SEQ ID NO: 23, 28 or 28.

In some embodiments, the 5' UTR is selected from an RNA sequence corresponding to the nucleic acid sequence set forth in SEQ ID NO: 3, 19 or 22, and the 3' UTR is selected from an RNA sequence corresponding to the nucleic acid sequence set forth in SEQ ID NO: 23, 24, 25, 28 or 29.

In some embodiments, the 5' UTR is selected from an RNA sequence corresponding to the nucleic acid sequence set forth in SEQ ID NO: 4, 12, 17, 20 or 21, and the 3' UTR is selected from an RNA sequence corresponding to the nucleic acid sequence set forth in SEQ ID NO: 23, 24, 28 or 29.

In some embodiments, the 5' UTR is an RNA sequence corresponding to the nucleic acid sequence set forth in SEQ ID NO: 19, and the 3' UTR is selected from an RNA sequence corresponding to the nucleic acid sequence set forth in SEQ ID NO: 24, 25, 28 or 29.

In a specific embodiment, the 5' UTR is an RNA sequence corresponding to the nucleic acid sequence set forth in SEQ ID NO: 3, and the 3' UTR is an RNA sequence corresponding to the nucleic acid sequence set forth in SEQ ID NO: 23.

In a specific embodiment, the 5' UTR is an RNA sequence corresponding to the nucleic acid sequence set forth in SEQ ID NO: 3, and the 3' UTR is an RNA sequence corresponding to the nucleic acid sequence set forth in SEQ ID NO: 24.

In a specific embodiment, the 5' UTR is an RNA sequence corresponding to the nucleic acid sequence set forth in SEQ ID NO: 3, and the 3' UTR is an RNA sequence corresponding to the nucleic acid sequence set forth in SEQ ID NO: 25.

In a specific embodiment, the 5' UTR is an RNA sequence corresponding to the nucleic acid sequence set forth in SEQ ID NO: 3, and the 3' UTR is an RNA sequence corresponding to the nucleic acid sequence set forth in SEQ ID NO: 28.

In a specific embodiment, the 5' UTR is an RNA sequence corresponding to the nucleic acid sequence set forth in SEQ ID NO: 3, and the 3' UTR is an RNA sequence corresponding to the nucleic acid sequence set forth in SEQ ID NO: 29.

In a specific embodiment, the 5' UTR is an RNA sequence corresponding to the nucleic acid sequence set forth in SEQ ID NO: 4, and the 3' UTR is an RNA sequence corresponding to the nucleic acid sequence set forth in SEQ ID NO: 23.

In a specific embodiment, the 5' UTR is an RNA sequence corresponding to the nucleic acid sequence set forth in SEQ ID NO: 4, and the 3' UTR is an RNA sequence corresponding to the nucleic acid sequence set forth in SEQ ID NO: 24.

In a specific embodiment, the 5' UTR is an RNA sequence corresponding to the nucleic acid sequence set forth in SEQ ID NO: 4, and the 3' UTR is an RNA sequence corresponding to the nucleic acid sequence set forth in SEQ ID NO: 28.

In a specific embodiment, the 5' UTR is an RNA sequence corresponding to the nucleic acid sequence set forth in SEQ ID NO: 4, and the 3' UTR is an RNA sequence corresponding to the nucleic acid sequence set forth in SEQ ID NO: 29.

In a specific embodiment, the 5' UTR is an RNA sequence corresponding to the nucleic acid sequence set forth in SEQ ID NO: 12, and the 3' UTR is an RNA sequence corresponding to the nucleic acid sequence set forth in SEQ ID NO: 23.

In a specific embodiment, the 5' UTR is an RNA sequence corresponding to the nucleic acid sequence set forth in SEQ ID NO: 12, and the 3' UTR is an RNA sequence corresponding to the nucleic acid sequence set forth in SEQ ID NO: 24.

In a specific embodiment, the 5' UTR is an RNA sequence corresponding to the nucleic acid sequence set forth in SEQ ID NO: 12, and the 3' UTR is an RNA sequence corresponding to the nucleic acid sequence set forth in SEQ ID NO: 28.

In a specific embodiment, the 5' UTR is an RNA sequence corresponding to the nucleic acid sequence set forth in SEQ ID NO: 12, and the 3' UTR is an RNA sequence corresponding to the nucleic acid sequence set forth in SEQ ID NO: 29.

In a specific embodiment, the 5' UTR is an RNA sequence corresponding to the nucleic acid sequence set forth in SEQ ID NO: 17, and the 3' UTR is an RNA sequence corresponding to the nucleic acid sequence set forth in SEQ ID NO: 23.

In a specific embodiment, the 5' UTR is an RNA sequence corresponding to the nucleic acid sequence set forth in SEQ ID NO: 17, and the 3' UTR is an RNA sequence corresponding to the nucleic acid sequence set forth in SEQ ID NO: 24.

In a specific embodiment, the 5' UTR is an RNA sequence corresponding to the nucleic acid sequence set forth in SEQ ID NO: 17, and the 3' UTR is an RNA sequence corresponding to the nucleic acid sequence set forth in SEQ ID NO: 25.

In a specific embodiment, the 5' UTR is an RNA sequence corresponding to the nucleic acid sequence set forth in SEQ ID NO: 17, and the 3' UTR is an RNA sequence corresponding to the nucleic acid sequence set forth in SEQ ID NO: 28.

In a specific embodiment, the 5' UTR is an RNA sequence corresponding to the nucleic acid sequence set forth in SEQ ID NO: 17, and the 3' UTR is an RNA sequence corresponding to the nucleic acid sequence set forth in SEQ ID NO: 29.

In a specific embodiment, the 5' UTR is an RNA sequence corresponding to the nucleic acid sequence set forth in SEQ ID NO: 19, and the 3' UTR is an RNA sequence corresponding to the nucleic acid sequence set forth in SEQ ID NO: 23.

In a specific embodiment, the 5' UTR is an RNA sequence corresponding to the nucleic acid sequence set forth in SEQ ID NO: 19, and the 3' UTR is an RNA sequence corresponding to the nucleic acid sequence set forth in SEQ ID NO: 24.

In a specific embodiment, the 5' UTR is an RNA sequence corresponding to the nucleic acid sequence set forth in SEQ ID NO: 19, and the 3' UTR is an RNA sequence corresponding to the nucleic acid sequence set forth in SEQ ID NO: 25.

In a specific embodiment, the 5' UTR is an RNA sequence corresponding to the nucleic acid sequence set forth in SEQ ID NO: 19, and the 3' UTR is an RNA sequence corresponding to the nucleic acid sequence set forth in SEQ ID NO: 28.

In a specific embodiment, the 5' UTR is an RNA sequence corresponding to the nucleic acid sequence set forth in SEQ ID NO: 19, and the 3' UTR is an RNA sequence corresponding to the nucleic acid sequence set forth in SEQ ID NO: 29.

In a specific embodiment, the 5' UTR is an RNA sequence corresponding to the nucleic acid sequence set forth in SEQ ID NO: 20, and the 3' UTR is an RNA sequence corresponding to the nucleic acid sequence set forth in SEQ ID NO: 23.

In a specific embodiment, the 5' UTR is an RNA sequence corresponding to the nucleic acid sequence set forth in SEQ ID NO: 20, and the 3' UTR is an RNA sequence corresponding to the nucleic acid sequence set forth in SEQ ID NO: 28.

In a specific embodiment, the 5' UTR is an RNA sequence corresponding to the nucleic acid sequence set forth in SEQ ID NO: 20, and the 3' UTR is an RNA sequence corresponding to the nucleic acid sequence set forth in SEQ ID NO: 29.

In a specific embodiment, the 5' UTR is an RNA sequence corresponding to the nucleic acid sequence set forth in SEQ ID NO: 21, and the 3' UTR is an RNA sequence corresponding to the nucleic acid sequence set forth in SEQ ID NO: 23.

In a specific embodiment, the 5' UTR is an RNA sequence corresponding to the nucleic acid sequence set forth in SEQ ID NO: 21, and the 3' UTR is an RNA sequence corresponding to the nucleic acid sequence set forth in SEQ ID NO: 24.

In a specific embodiment, the 5' UTR is an RNA sequence corresponding to the nucleic acid sequence set forth in SEQ ID NO: 21, and the 3' UTR is an RNA sequence corresponding to the nucleic acid sequence set forth in SEQ ID NO: 28.

In a specific embodiment, the 5' UTR is an RNA sequence corresponding to the nucleic acid sequence set forth in SEQ ID NO: 22, and the 3' UTR is an RNA sequence corresponding to the nucleic acid sequence set forth in SEQ ID NO: 24.

In a specific embodiment, the 5' UTR is an RNA sequence corresponding to the nucleic acid sequence set forth in SEQ ID NO: 22, and the 3' UTR is an RNA sequence corresponding to the nucleic acid sequence set forth in SEQ ID NO: 25.

In a specific embodiment, the 5' UTR is an RNA sequence corresponding to the nucleic acid sequence set forth in SEQ ID NO: 22, and the 3' UTR is an RNA sequence corresponding to the nucleic acid sequence set forth in SEQ ID NO: 28. Or
In a specific embodiment, the 5' UTR is an RNA sequence corresponding to the nucleic acid sequence set forth in SEQ ID NO: 22, and the 3' UTR is an RNA sequence corresponding to the nucleic acid sequence set forth in SEQ ID NO: 29.

In some embodiments, the mRNA molecule further comprises a nucleic acid sequence encoding a target polypeptide.

In some embodiments, the mRNA molecule further comprises polyA.

**In one aspect,** the present disclosure provides a method for increasing the protein expression level or translation efficiency of mRNA using the 5' UTR and 3' UTR combinations or mRNA molecules comprising 5' UTR and 3' UTR as defined above.

**In one aspect,** the present disclosure provides the 5' UTR and 3' UTR combinations or mRNA molecules comprising 5' UTR and 3' UTR as defined above for use in increasing the protein expression level or translation efficiency of mRNA.

**In one aspect,** the present disclosure provides use of the 5' UTR and 3' UTR combinations as defined above in increasing the protein expression level or translation efficiency of mRNA.

**In one aspect,** the present disclosure provides a DNA encoding the aforementioned mRNA molecule.

**In one aspect,** the present disclosure provides a vector comprising the aforementioned DNA.

**In one aspect,** the present disclosure provides a host cell comprising the aforementioned vector.

**In one aspect,** the present disclosure provides a lipid nanoparticle comprising the aforementioned mRNA molecule.

**In one aspect,** the present disclosure provides a pharmaceutical composition comprising the aforementioned mRNA molecule, the aforementioned DNA, the aforementioned vector, the aforementioned host cell or the aforementioned lipid nanoparticle, and a pharmaceutically acceptable carrier.

**In one aspect,** the present disclosure provides a method of performing gene therapy, gene vaccination, protein replacement therapy, antisense therapy, or therapy by interfering RNA using the aforementioned mRNA molecule, the aforementioned DNA, the aforementioned vector, the aforementioned host cell, the aforementioned lipid nanoparticle or the aforementioned pharmaceutical composition.

**In one aspect,** the present disclosure provides the aforementioned mRNA molecule, the aforementioned DNA, the aforementioned vector, the aforementioned host cell, the aforementioned lipid nanoparticle or the aforementioned pharmaceutical composition for use in gene therapy, gene vaccination, protein replacement therapy, antisense therapy, or therapy by interfering RNA.

**In one aspect,** the present disclosure provides use of the aforementioned mRNA molecule, the aforementioned DNA, the aforementioned vector, the aforementioned host cell, the aforementioned lipid nanoparticle or the aforementioned pharmaceutical composition in the preparation of a drug for gene therapy, gene vaccination, protein replacement therapy, antisense therapy, or therapy by interfering RNA.

### DESCRIPTION OF DRAWINGS

FIG. 1 shows the effect of different 5' UTRs on GFP expression level.
FIG. 2 shows the effect of different 3' UTRs on GFP expression level.

### DETAILED DESCRIPTION OF THE INVENTION

Although the present invention is described in detail below, it should be understood that the present invention is not limited to the particular methods, protocols and reagents described herein, as these may be altered. It is further understood that the terms used herein are intended solely to describe the purpose of some particular embodiments and not to limit the scope of the present invention which will be limited only by the appended claims. Unless otherwise defined, all technical and scientific terms used herein have the same meanings as are generally understood by those of ordinary skill in the art.

Unless otherwise indicated, the implementation of the present invention will employ conventional chemical, biochemical, cellular biological, immunological and recombinant DNA techniques as described in the literature in the art (see e.g., Molecular Cloning: A Laboratory Manual, 2nd ed., J. Sambrook et al., eds., Cold Spring Harbor Laboratory Press, Cold Spring Harbor 1989).

Unless the context otherwise requires, the words "comprising/including" and variations thereof throughout the specification and the appended claims shall be understood to imply the inclusion of stated elements, integers or steps or groups of elements, integers or steps but not the exclusion of any other elements, integers or steps or groups of elements, integers or steps. Unless otherwise indicated herein or clearly contradicted by the context, nouns without quantifier used in the context of describing the present invention (especially in the context of claims) should be interpreted as indicating one and/or more. The description of a range of values herein is intended only as a shorthand for separate references to individual values falling within the range. Unless otherwise noted herein, individual values are incorporated into the specification as if they were separately recorded herein. Unless otherwise indicated herein or clearly contradicted by the context, all methods described herein may be performed in any suitable order. The use of any and all examples or exemplary languages (e.g., "for example/such as") provided herein is intended only to better illustrate the present invention, without limiting the scope of the present invention otherwise claimed. No language in the specification should be construed as indicating any unclaimed element necessary for the implementation of the present invention.

According to the present invention, a nucleic acid molecule or a nucleic acid sequence refers to a nucleic acid, which is preferably deoxyribonucleic acid (DNA) or ribonucleic acid (RNA). According to the present invention, nucleic acids comprise genomic DNA, cDNA, mRNA, recombinant molecules and chemically synthesized molecules. According to the present invention, nucleic acids may be in the form of a linear or covalently closed cyclic molecule of a single or double chain.

In the context of the present invention, the term "RNA" refers to a molecule comprising ribonucleotide residues, and preferably consisting entirely or substantially of ribonucleotide residues. The term "ribonucleotide" refers to a nucleotide having a hydroxyl group at the 2' position of the β-D-ribofuranosyl group. The term "RNA" includes double-stranded RNA, single-stranded RN, isolated RNA (e.g., partially or fully purified RNA), substantially pure RNA, synthetic RNA, and recombinantly produced RNA, e.g., modified RNA, which differs from naturally occurring RNA in that one or more nucleotides are added, deleted, substituted, and/or altered. Such changes may include adding a non-nucleotide material, such as to the ends of RNA or internally, for example, adding a non-nucleotide material at one or more nucleotides of RNA. The nucleotides in the RNA molecule may also comprise non-standard nucleotides, such as non-naturally occurring nucleotides or chemically synthesized nucleotides or deoxynucleotides. These altered RNAs can be referred to as analogs, particularly analogs of naturally occurring RNAs. According to the present invention, RNA includes mRNA.

The term "mRNA" means "messenger RNA" and relates to transcripts that are produced by using DNA templates and encode peptides or proteins. In general, mRNA comprises 5' UTR, a protein coding region, 3' UTR, and a poly (A) sequence. mRNA can be produced by transcription of DNA templates in vitro. In vitro transcription methods are known to technicians. For example, various in vitro transcription kits are commercially available. According to the present invention, in addition to the modifications according to the present invention, mRNA can also be modified by further stabilization modification and capping.

The term "nucleic acid" according to the present invention also includes chemical derivatization of nucleic acid on nucleotide bases, on saccharides or on phosphoric acid, and nucleic acids containing non-naturally occurring nucleotides and nucleotide analogs.

"Fragment" or "fragment of a nucleic acid sequence" relates to a portion of a nucleic acid sequence, i.e., a sequence that appears as a nucleic acid sequence shortened at the 5' and/or 3' ends. Preferably, when the fragment replaces the nucleic acid sequence in the RNA molecule, it retains RNA stability and/or translation efficiency. Preferably, a fragment of a nucleic acid sequence comprises at least 80%, preferably at least 90%, 95%, 96%, 97%, 98% or 99% of the nucleotide residues from the nucleic acid sequence.

The term "variant" in accordance with the present invention for, for example, nucleic acid and amino acid sequences, includes any variant, in particular mutants, splicing variants, conformers, isomers, allelic variants, species variants, and species homologues, especially those that are naturally occurring. Allelic variants involve changes in the normal sequence of a gene, the importance of which is often unclear. Complete gene sequencing often identifies a large number of allelic variants of a given gene. Species homologues are nucleic acid or amino acid sequences that have different species origins from a given nucleic acid or amino acid sequence.

According to the present invention, a nucleic acid variant comprises single or multiple nucleotide deletions, additions, mutations and/or insertions as compared to a reference nucleic acid. Deletion involves removing one or more nucleotides from a reference nucleic acid. The addition variant comprises 5' and/or 3' end fusion of one or more nucleotides (e.g., 1, 2, 3, 5, 10, 20, 30, 50 or more nucleotides). Mutations may include, but are not limited to, substitutions, wherein at least one nucleotide in a sequence is removed and another nucleotide is inserted in its position (e.g., transversions and transitions); base-free sites; cross-linking sites; and chemically altered or modified bases. Insertion involves adding at least one nucleotide to a reference nucleic acid.

For nucleic acid molecules, the term "variant" includes a degenerate nucleic acid sequence, wherein the degenerate nucleic acid sequence according to the present invention is a nucleic acid different from a reference nucleic acid in a codon sequence due to degeneracy of a genetic code.

Preferably, the degree of identity between a given nucleic acid sequence and a nucleic acid sequence that is a variant of the given nucleic acid sequence is at least 70%, preferably at least 75%, preferably at least 80%, more preferably at least 85%, more preferably at least 90%, or most preferably at least 95%, 96%, 97%, 98%, or 99%. The degree of identity is preferably given for regions having at least about 30, at least about 50, at least about 70, at least about 90, at least about 100, at least about 150, at least about 200, at least about 250, at least about 300 or at least about 400 nucleotides. In some preferred embodiments, the degree of identity is given for the entire length of the reference nucleic acid sequence.

"Sequence similarity" refers to the percentage of amino acids that are identical or represent conservative amino acid substitutions. The "sequence identity" between two polypeptide or nucleic acid sequences represents the percentage of amino acids or nucleotides that are identical between these sequences.

The term "% identity" is intended to refer to the percentage of identical nucleotides, in particular in the optimal alignment between two sequences to be compared, wherein the percentage is purely statistical and the differences between the two sequences can be randomly distributed over the full length of the sequence, and the sequence to be compared may comprise additions or deletions to obtain the optimal alignment between the two sequences compared to the reference sequence. The comparison of two sequences is typically done by comparing the sequences after the optimal alignment for a segment or "comparison window" to identify local regions of the corresponding sequence. The optimal alignment for comparison can be performed manually, or with the aid of the local homology algorithm of Smith and Waterman, 1981, Ads App. Math. 2, 482, with the aid of the local homology algorithm of Neddleman and Wunsch, 1970, J. Mol. Biol. 48, 443, and with the aid of the similarity search algorithm of Pearson and Lipman, 1988, Proc. Natl Acad. Sci. USA 85, 2444, or with the aid of computer programs that use such algorithms (GAP, BESTFIT, FASTA, BLAST P, BLAST N, and TFASTA in the Wisconsin Genetics Software Package, Genetics Computer Group, 575 Science Drive, Madison, Wis.).

The percentage identity is obtained by determining the number of identical positions corresponding to the sequence to be compared, dividing the number by the number of compared positions, and multiplying the result by 100. For example, the BLAST program "BLAST 2 sequences", which is available on the website http://www.ncbi.nlm.nih.gov/blast/bl2seq/wblast2.cgi, can be used.

All of the successive residues of an acid sequence will be hydrogen bonded to the same number of successive residues in the second nucleic acid sequence. Preferably, the degree of complementarity according to the present invention is at least 70%, preferably at least 75%, preferably at least 80%, more preferably at least 85%, even more preferably at least 90%, or most preferably at least 95%, 96%, 97%, 98% or 99%. Most preferably, the degree of complementarity according to the present invention is 100%.

A fragment or variant of a specific nucleic acid sequence or a nucleic acid sequence having a specific degree of identity with a specific nucleic acid sequence preferably has at least one functional characteristic of the specific sequence and preferably is functionally equivalent to the specific sequence, which is, for example, a nucleic acid sequence exhibiting a characteristic identical to or similar to that of the specific nucleic acid sequence.

The term "promoter" or "promoter region" refers to a DNA sequence upstream (5') of the coding sequence of a gene, which controls the expression of the coding sequence by providing recognition and binding sites for RNA polymerase. The promoter region may comprise additional recognition or binding sites of other factors involved in regulating the transcription of the gene. Promoters can control the transcription of prokaryotic or eukaryotic genes. The promoter may be "inducible" and initiate transcription in response to an inducer, or may be "constitutive" if transcription is not controlled by the inducer. Without inducers, inducible promoters are expressed only to a small extent or not expressed at all. In the presence of an inducer, the gene "turns on" or has increased transcription level. This is usually mediated by binding of specific transcription factors.

According to the present invention, the term "expression" is used in its most general sense and includes the production of RNA or RNA and proteins. It also includes partial expression of nucleic acids. In addition, expression may be transient or stable. With respect to RNA, the term "expression" or "translation" relates to a process in the ribosome of a cell in which the strand of a messenger RNA guides the assembly of an amino acid sequence to produce a peptide or protein.

In the context of the present invention, the term "transcription" relates to a process in which the genetic code in a DNA sequence is transcribed into RNA. Subsequently, RNA can be translated into proteins. In accordance with that invention, the term "transcription" comprises "in vitro transcription", wherein the term "in vitro transcription" relates to a process in which RNA, in particular mRNA, is synthesized in vitro in a cell-free system. Preferably, cloning vectors are applied to the production of transcripts. These cloning vectors are generally named transcription vectors and are covered by the term "vector" according to the present invention. According to the present invention, RNA is preferably an in vitro transcribed RNA (IVT-RNA) and can be obtained by in vitro transcription of a suitable DNA template. The promoter for controlling transcription may be any promoter of any RNA polymerase. DNA templates for in vitro transcription can be obtained by cloning nucleic acids (in particular cDNA) and introducing them into suitable vectors for in vitro transcription. cDNA can be obtained by reverse transcription of RNA.

The term "untranslated region" or "UTR" used according to the present invention relates to mRNA portions upstream of start codon and downstream of stop codon, which are not translated and are therefore referred to as 5' untranslated region (5' UTR) and 3' untranslated region (3' UTR), respectively. These regions are transcribed with coding regions and are therefore exon-like when present in mature mRNA.

3'-untranslated region (3' UTR): In general, the term "3' UTR" refers to the portion of an artificial nucleic acid molecule that is located at 3' (i.e., "downstream") of an open reading frame and is not translated into a protein. Typically, 3' UTR is the portion of mRNA between a protein coding region (open reading frame (ORF) or coding sequence (CDS)) and a polyadenylate sequence of mRNA. In the context of the present invention, the term 3' UTR may also comprise elements, such as polyadenylate sequences, which are not encoded in the template from which the RNA is transcribed but which are added during post-transcriptional maturation. The 3'-UTR of mRNA is not translated into an amino acid sequence. 3'-UTR sequences are usually encoded by genes that are transcribed into their respective mRNAs during gene expression. The genome sequence is first transcribed into a pre-mRNA comprising an optional intron. The pre-mRNA is then further processed into mature mRNA during the maturation process. The maturation process comprises the steps of: 5' capping, splicing pre-mRNA to remove optional introns and 3' end modifications (e.g., polyadenylation of the 3' end of the pre-mRNA and optional endonuclease/or exonuclease cleavage). Within the scope of the present invention, 3'-UTR corresponds to the sequence of a mature mRNA between the stop codon of the protein coding region, preferably immediately adjacent to the 3' end of the stop codon of the protein coding region, and the polyadenylate sequence of the mRNA. The term "correspond to" means that the 3'-UTR sequence may be, for example, an RNA sequence in the mRNA sequence used to define the 3'-UTR sequence, or a DNA sequence corresponding to the RNA sequence. Within the scope of the present invention, the term "3'-UTR of a gene", such as "3'-UTR of a ribosomal protein gene", is a sequence corresponding to a 3'UTR derived from a mature mRNA of the gene, the mature mRNA being the mRNA obtained by gene transcription and maturation of the pre-mRNA. The term "3'UTR of a gene" includes the DNA and RNA sequences of 3'UTR (both sense and antisense and both mature and immature).

5'-untranslated region (5' UTR): 5' UTR is typically understood as a specific part of messenger RNA (mRNA). It is located at the 5' side of the mRNA open reading frame. Typically, 5' UTR begins at the transcription start site and ends at the nucleotide preceding the open reading frame start codon. 5' UTR can comprise elements that control gene expression, also known as regulatory elements. Such regulatory element may be, for example, ribosome binding sites. 5' UTR may be post-transcriptionally modified, for example, by adding 5'-cap. Within the scope of the present invention, 5' UTR corresponds to a mature mRNA sequence located between the 5' cap and the start codon. Preferably, 5' UTR corresponds to a sequence extending from a nucleotide at the 3' side of the 5'-cap, preferably from a nucleotide immediately adjacent to the 3' side of the 5'-cap, to a nucleotide at the 5' side of the start codon in the protein coding region, preferably to a nucleotide immediately adjacent to the 5' side of the start codon in the protein coding region. Nucleotides immediately adjacent to the 3' side of the mature mRNA5'-cap typically correspond to transcription start sites. The term "correspond to" means that the 5'-UTR sequence may be, for example, an RNA sequence in the mRNA sequence used to define the 5'-UTR sequence, or a DNA sequence corresponding to the RNA sequence. Within the scope of the present invention, the term "5'-UTR of a gene" is a sequence corresponding to a 5'UTR derived from a mature mRNA of the gene, the mature mRNA being the mRNA obtained by gene transcription and maturation of the pre-mRNA. The term "5' UTR of a gene" includes the DNA sequence and RNA sequence of the 5' UTR.

According to the present invention, the term "gene" refers to a specific nucleic acid sequence responsible for producing one or more cell products and/or for performing one or more intercellular or intracellular functions. More particularly, the term relates to DNA segments comprising nucleic acids encoding specific proteins or functional or structural RNA molecules.

Polyadenylation involves adding a poly(A) sequence (PolyA) or a poly(A) tail to the primary transcript RNA. The poly(A) sequence consists of a plurality of adenosine monophosphates. In other words, it is a segment of RNA with only adenine bases. In eukaryotes, polyadenylation is part of the process of producing mature messenger RNA (mRNA) for translation. Thus, it forms part of a larger gene expression process. Polyadenylation begins at the end or termination of gene transcription. The most 3' segment of the newly generated pre-mRNA is first cut off by a set of proteins; these proteins then synthesize the poly(A) sequence at the 3' end of the RNA. Poly(A) sequences are important for nuclear export, translation and mRNA stability. This sequence is shortened over time and when it is sufficiently short, mRNA is enzymatically degraded.

The term "polyadenylate sequence", "poly(A) sequence" or "poly(A) tail" refers to the sequence of adenylate residues normally at the 3' end of the RNA molecule. The invention allows such sequences to be attached during RNA transcription via a DNA template based on repeated thymidylate residues in the strands complementary to the coding strands, however the sequences are not normally encoded in DNA but are attached to the free 3' end of RNA by template-independent RNA polymerase after transcription in the nucleus. According to the present invention, in an embodiment, the poly(A) sequence has at least 20, preferably at least 40, preferably at least 80, preferably at least 100 and preferably up to 500, preferably up to 400, preferably up to 300, preferably up to 200 and in particular up to 150 A nucleotides, preferably consecutive A nucleotides, and in particular about 120 A nucleotides. The term "A nucleotide" or "A" refers to adenylate residues.

The nucleic acids described herein may be recombinant molecules and/or isolated molecules.

The term "isolated molecule" used herein is intended to refer to molecules that are substantially free of other molecules, such as other cellular substances. The term "isolated nucleic acid" means, according to the present invention, that the nucleic acid has been: (i) amplified in vitro, e.g., by polymerase chain reaction (PCR) in vitro; (ii) produced by cloning recombination; (iii) purified, e.g., by cleavage and gel electrophoresis fractionation; or (iv) synthesized, e.g., by chemical synthesis. Isolated nucleic acids are nucleic acids that can be manipulated by recombinant DNA techniques.

The term "recombinant" in the context of the present invention means "produced by genetic engineering". Preferably, in the context of the present invention, "recombinant substances" such as recombinant cells are not naturally occurring.

The term "naturally occurring" used herein refers to the fact that a substance is found in nature. For example, peptides or nucleic acids that are present in organisms (including viruses) and that can be isolated from natural sources and have not been artificially modified in experiments are naturally occurring.

According to the present invention, the term "host cell" refers to any cell that can be transformed or transfected with an exogenous nucleic acid. In accordance with the present invention, the term "host cell" comprises prokaryotic cells (e.g., Escherichia coli) or eukaryotic cells (e.g., yeast cells and insect cells). Mammalian cells, such as cells from humans, mice, hamsters, pigs, goats, primates, are particularly preferred. Cells may originate from a variety of tissue types and include primary cells and cell lines. Some specific examples include keratinocytes, peripheral blood leukocytes, bone marrow stem cells and embryonic stem cells. In still other embodiments, the host cell is an antigen presenting cell, particularly a dendritic cell, a monocyte or a macrophage. Nucleic acids may be present in the host cells in a single copy or in several copies, and in an embodiment, they are expressed in the host cells.

According to the present invention, the term "peptide" comprises oligopeptides and polypeptides, and refers to substances comprising 2 or more, preferably 3 or more, preferably 4 or more, preferably 6 or more, preferably 8 or more, preferably 10 or more, preferably 13 or more, preferably 16 or more, preferably 20 or more, and preferably up to 50, preferably 100 or preferably 150 consecutive amino acids, which are linked to each other by peptide bonds. The term "protein" refers to a macropeptide, preferably a peptide having at least 151 amino acids, but generally the terms "peptide" and "protein" are used herein as synonyms.

The terms "peptide" and "protein" according to the present invention comprise substances containing not only amino acid components but also non-amino acid components such as saccharide or phosphate structures, and further comprise substances containing a bond such as an ester bond, a thioether bond or a disulfide bond.

According to the present invention, nucleic acids, such as RNA, may encode peptides or proteins. Thus, the transcriptable nucleic acid sequence or transcript thereof may comprise an open reading frame (ORF) encoding a peptide or protein. The nucleic acid can express the encoded peptide or protein. For example, the nucleic acid may be a nucleic acid encoding and expressing an antigen or a pharmaceutically active peptide or protein such as an immunoactive compound, which is preferably not an antigen.

According to the present invention, the term "nucleic acid encoding a peptide or protein" means that if present in an appropriate environment, preferably in a cell, the nucleic acid can direct the assembly of amino acids during translation to produce a peptide or protein. Preferably, the RNA according to the present invention is capable of interacting with a cell translation mechanism to allow peptide or protein translation.

**In one aspect,** the inventors of the present application screen out 5'-UTR and 3'-UTR combinations capable of significantly increasing the expression level of mRNA. As confirmed by embodiments of the present application, a variety of 5' UTR and 3' UTR combinations are unexpectedly capable of significantly increasing the protein expression level of mRNA.

More specifically, when the 5' UTR is selected from an RNA sequence corresponding to the nucleic acid sequence set forth in SEQ ID NO: 3, 4, 12, 17, 19, 20, 21 or 22, and the 3' UTR is selected from an RNA sequence corresponding to the nucleic acid sequence set forth in SEQ ID NO: 23, 24, 25, 28 or 29, the 5' UTR and 3' UTR combination can significantly improve the translation efficiency of the coding sequence of a target gene, thereby significantly increasing the expression level thereof.

Therefore, in some embodiments, the 5' UTR is selected from an RNA sequence corresponding to the nucleic acid sequence set forth in SEQ ID NO: 3, 4, 12, 17, 19, 20, 21 or 22.

In some embodiments, the 3' UTR is selected from an RNA sequence corresponding to the nucleic acid sequence set forth in SEQ ID NO: 23, 24, 25, 28 or 29.

In some embodiments, the 5' UTR is selected from an RNA sequence corresponding to the nucleic acid sequence set forth in SEQ ID NO: 19 or 22, and the 3' UTR is selected from an RNA sequence corresponding to the nucleic acid sequence set forth in SEQ ID NO: 23, 24, 25, 28 or 29.

In some embodiments, the 5' UTR is selected from an RNA sequence corresponding to the nucleic acid sequence set forth in SEQ ID NO: 3, 4 or 12, and the 3' UTR is selected from an RNA sequence corresponding to the nucleic acid sequence set forth in SEQ ID NO: 23, 24, 28 or 29.

In some embodiments, the 5' UTR is an RNA sequence corresponding to the nucleic acid sequence set forth in SEQ ID NO: 17, and the 3' UTR is selected from an RNA sequence corresponding to the nucleic acid sequence set forth in SEQ ID NO: 23, 24 or 28.

In some embodiments, the 5' UTR is selected from an RNA sequence corresponding to the nucleic acid sequence set forth in SEQ ID NO: 20 or 21, and the 3' UTR is selected from an RNA sequence corresponding to the nucleic acid sequence set forth in SEQ ID NO: 23, 28 or 28.

In some embodiments, the 5' UTR is selected from an RNA sequence corresponding to the nucleic acid sequence set forth in SEQ ID NO: 3, 19 or 22, and the 3' UTR is selected from an RNA sequence corresponding to the nucleic acid sequence set forth in SEQ ID NO: 23, 24, 25, 28 or 29.

In some embodiments, the 5' UTR is selected from an RNA sequence corresponding to the nucleic acid sequence set forth in SEQ ID NO: 4, 12, 17, 20 or 21, and the 3' UTR is selected from an RNA sequence corresponding to the nucleic acid sequence set forth in SEQ ID NO: 23, 24, 28 or 29.

In some embodiments, the 5' UTR is an RNA sequence corresponding to the nucleic acid sequence set forth in SEQ ID NO: 19, and the 3' UTR is selected from an RNA sequence corresponding to the nucleic acid sequence set forth in SEQ ID NO: 24, 25, 28 or 29.

In some embodiments, the 5' UTR and 3' UTR combination does not include RNA sequences corresponding to the nucleic acid sequences set forth below: SEQ ID NO: 22 and SEQ ID NO: 23, SEQ ID NO: 20 and SEQ ID NO: 24, and SEQ ID NO: 21 and SEQ ID NO: 25.

In some embodiments, the mRNA molecule further comprises a nucleic acid sequence encoding a target polypeptide.

In some embodiments, the mRNA molecule further comprises polyA.

In some embodiments, the mRNA may be advantageously used in gene therapy, gene vaccination, protein replacement therapy, antisense therapy, or therapy by interfering RNA.

**In one aspect,** the present disclosure provides use of the 5' UTR and 3' UTR combinations as defined above in increasing the protein expression level or translation efficiency of mRNA.

Unless expressly stated otherwise in the context, the singular terms used herein "a", "an" and "the" are also intended to include the plural referents thereof. In addition, the open expressions "including" and "comprising" may be interpreted to also contain structural components or methodological steps that are not addressed, but it is important to note that the open expression also covers situations consisting solely of the components and methodological steps described (i.e., covers situations of closed expression "consisting of").

In general, the term "about" is used herein to refer to a change in the numerical value within 5% above and below a stated value.

Exemplary 5' UTR and 3' UTR sequences of the present application are shown in Table 1.

**Table 1. Exemplary 5' UTR and 3' UTR sequences**

| **Sequence No.** | **Name** | **Sequence** |
|---|---|---|
| SEQ ID NO: 1 | UTR5_4 | CCTTAAAG |
| SEQ ID NO: 2 | UTR5_5 | TTAAATT |
| SEQ ID NO: 3 | UTR5_3 0 | GATTGCTTATAGACCGGAAGCCGGGACCTTAATTC TCTTTCCCATCTTGCAAG |
| SEQ ID NO: 4 | UTR5_3 2 | CTGAACTGGGTTAGGTGCCGCTGTTGCTGCTCGTGT TGAATCTAGAACCGTAGCCAGAC |
| SEQ ID NO: 5 | UTR5_4 1 | AAAATTTTGCAGGGTAAATTAGCCTGTCTGGGTCT GAGCTCTTTATTCTTGGATACGTTGGAGATCGGAA AGATATTAAGAT |
| SEQ ID NO: 6 | UTR5_4 5 | AGTATATTAGTGCTAATTTCCCTCCGTTTGTCCTAG CTTTTCTCTTCTGTCAACCCCACACGCCTTTGGCAC A |
| SEQ ID NO: 7 | UTR5_4 6 | CGGCGCGCCGGGCTTCGCGCTCCGGTACTTCCTATT TAACAGACATTGTGTGGTGGGCTGGCACAATCGCA |
| SEQ ID NO: 8 | UTR5_5 0 | TTCCTGTCCCGCTCGGCTCCAGGCCCTGCGTGTCCC AGGGACTGCGGCCGGGGCGGGGTGGGGCTCTCCCC TGGCCAGGA |
| SEQ ID NO: 9 | UTR5_5 2 | TTCTCCCCCAGGCTCACTCACC |
| SEQ ID NO: 10 | UTR5_5 3 | GCCAGGCCGGCCAGGCCTGCCGCCGCCGCCGCC GCCGTCGCCGCCGCGCCGACC |
| SEQ ID NO: 11 | UTR5_5 7 | AACGGGGGCGCGCGGTCGGGGGCAGGTTGGGCCG GAAGAGGGGTCCGGGCCGGCACTGAGGCGGGAAG GGGGTCGGTTGAG |
| SEQ ID NO: 12 | UTR5_5 9 | AGTTCCCTATCACTCTCTTTAATCACTACTCACAGT AACCTCAACTCCTGCCACA |
| SEQ ID NO: 13 | UTR5_6 3 | GCTGGTTTTGGTGATTTTTGTCTGATTGCA |
| SEQ ID NO: 14 | UTRS 6 6 | GCCTCTTCCCCCTCCCGCGCGCCCGCCCGCCGCCTG CCGCCGCCGCCGCCGCCGCCGGAGCTCTGTAGT |
| SEQ ID NO: 15 | UTR5_6 9 | TGTGAGTTCATTCCTCCCTGCTCAGATTCTTCAGCT CCTCGTGGTTGGTTTATTCAATTGGTTGTTTCATT |
| SEQ ID NO: 16 | UTR5_7 3 | CTCCTTCCCTCGTCCTGGGTCCCGGGGTCCTGGGTA CGTTTGGCTAGCCTGCTCTGGCGGTGCAGAGGGGC AGG |
| SEQ ID NO: 17 | UTR5_7 9 | ATTTTGTTTTTGTGTTTTTGCAGGGAGGAGCTCTTC CTGCAGGCGTGGAAACC |
| SEQ ID NO: 18 | UTR5_8 1 | AGAGAAAT |
| SEQ ID NO: 19 | UTR5_8 3 | ATTAAGGAGAAAAAGTAGGAGGAGGAGGAGAAAG AGTGCTTTGCCCAGGGTGTGACTGAAGAAATATCA A |
| SEQ ID NO: 20 | UTR5_9 1 | GAAATAAGAGAGAAAAGAAGAGTAAGAAGAAATA TAAGA |
| SEQ ID NO: 21 | UTR5_9 2 | GAATAAACTAGTATTCTTCTGGTCCCCACAGACTC AGAGAGAACCC |
| SEQ ID NO: 22 | 028M | AAGCTTAGCTTGCTTGTTCTTTTTGCAGAAGCTCAG AATAAACGCTCAACTTTGGCGCC |
| SEQ ID NO: 23 | UTR3_1 | TAGGCTGGAGCCTCGGTGGCCATGCTTCTTGCCCCT TGGGCCTCCCCCCAGCCCCTCCTCCCCTTCCTGCAC CCGTACCCCCGTGGTCTTTGAATAAAGTCTGAGTG GGCGGC |
| SEQ ID NO: 24 | UTR3_5 | GCTGGAGCCTCGGTGGCCATGCTTCTTGCCCCTTGG GCCTCCCCCCAGCCCCTCCTCCCCTTCCTGCACCCG TACCCCCCAAACACCATTGTCACACTCCAGTGGTCT TTGAATAAAGTCTGAGTGGGCGGC |
| SEQ ID NO: 25 | UTR3_7 | CTGGTACTGCATGCACGCAATGCTAGCTGCCCCTTT CCCGTCCTGGGTACCCCGAGTCTCCCCCGACCTCG GGTCCCAGGTATGCTCCCACCTCCACCTGCCCCACT CACCACCTCTGCTAGTTCCAGACACCTCCCAAGCA CGCAGCAATGCAGCTCAAAACGCTTAGCCTAGCCA CACCCCCACGGGAAACAGCAGTGATTAACCTTTAG CAATAAACGAAAGTTTAACTAAGCTATACTAACCC CAGGGTTGGTCAATTTCGTGCCAGCCACACCCTGG AGCTAGC |
| SEQ ID NO: 26 | UTR3_8 | CTGCCTCTCGCTCCTCAACCCCTCCCCTCCATCCCT GGCCCCCTCCCTGGATGACATTAAAGAAGGGTTGA GCTGGTCCCTGCCTGCATGTGACTGTAAA |
| SEQ ID NO: 27 | UTR3_1 8 | ATAAAATGAGAAAGGTATGTTTTTCTTTTTTAAAAA AGCTGCTGTTGGATCTAGAAGGTGAAGAATTTTTTT ATGTATATATAGACATATCTATATAAATTGTCTGGC TGAGGCAGGGCCTTCAGCTATCATTTGGTTAATAA ATACATTTTAGTATTTGCATTTCCTACTGCCTGCAG AGTTTCAGGTGCTTGTTGTGTGAAAGTTCTGTAGAT GTGTGCAAATTTAACGAAATGAAATTGTATGTGTA AAAATGTACGATTTTTCACTGTGCAACTGTAAATTA TAAATAAAAAATATTTTTGCTATTCATGGAGTGTA ATATTTATGCACACCATCAAATAGTTTCTGTACTTT TTATTGGGTAAAAATGGAATTGAACAGCAACCTCA ACATAAGATTTTTTTTCTAGTAGCCTCCCACTGATT AAAGAAGCAAGTTTGAGGTTTCATCCTTCAAAAGG GGGTTCCGAGAGAGCACCGTAGGGCTTTTCTCAAA TAGAAAAGCCAGATTTTGAAAAAATTTTAAAGATA AAATAGGACATATTTTGCAGATATATATATATATA TACACAAACACATCTCCAGGTATAGAGAACCATCC AGATGTTCACTTTTGAAAATATCTAATGATGCAAA GTTTTATTCTTGAACTTGGACACTGATGCCATCAAA CAATTAACAAATATATTTAAGTACTAAAGGTGATT TTTTTTTTAAAGACTTTTTCAAATTGTCAAATGATT TAATGCAGATGAACATATTTCTATTTTAAGTAACG GGAATCTGTAAGAATGTTTGCTTGAGATATGGTTA ACTTTTTTCTTTTGTTGGTTTTGACTTAGATGGACA CCATGAGATGTTAATATTCATACATGTAATAAATA GAATGATGAAG |
| SEQ ID NO: 28 | UTR3_3 2 | TCCTTTAAGGGAGTGAGGTGCTGAGGCCTGGTGGC AGAGGCAGCCCTGGCTCCCCCATGGCCACTGTGTC CTGCTGGGAAGGAGGGCCTGGCTCCACGACCCACC TCTGCCGGCCTGGGGCT |
| SEQ ID NO: 29 | UTR3_3 4 | CACCCAGGCCTGGAGAAGATGAGACACCTGGGCCC ATCTGGCTGCTGGAGATTCAGTCTCAGTTTTATTTC TCTGTGGCACTCACTGCTTCTGGAGGGGAGACTGT TAATAAAAGATTTGGGAACCCCC |
| SEQ ID NO: 30 | UTR3_4 3 | GCAGCTGGGGTGCCTCCCTCGGGTGTCCAGCAAGC ACTGGGGAGTCCCCCTTAGGGCTGGCTTTGAGATA GATCCTGATGTCAGCCTGTTTCACAGATGAGACAA TGGAGGCTTTGATCGCTTGCCCAGGGCCACGTCCA GTGAGTGGTACAGCTTGGACTCAAACCCAAGTCTG TGTGACTCCAACATGTCACACTTCCTCACGTGAAA AGCCTCATATGAGGCTGGGCACAGTGACTTGTGCC TGTAATCCCAGCACTTTGG |
| SEQ ID NO: 31 | UTR3_7 6 | TGTTCTGCCAGGAACACTGCTTCCTGCCAGGTACCC ACAGACCATGGGAGCTTAGGTTCTGTGTGTCTGGA ATGTTTTCTGCTGCTTCTCTGCTCACATGGTTGGGG GGATGCAGGGGTTGTGAGGGAAGGCACTGCTTATT GCCACCTTCCAGTGAATGC |
| SEQ ID NO: 32 | UTR3_7 7 | TTAGGGAGTCTGCCCTTTTTCTGTGCCCTGGGACCT GAGCATGTGGGAGCAGGGCAGATGGGTGGCAGGA GGCCAGGGGTTGGATCATGTTCCCCCCAAATGCTG GGAAGCAATAAGCCTCCTCCCCCCCACCATTTCT CTGGAGATTAGGACTTATTCTCACAGCTGGAGGAA GCTCAACAACTTTCCTGAGGACAATGATTGTTTGA AAGGCTTGTTTTGTAAGGAAGCCAGGAGGAATATA TGAGTTTGATGAGCTAAAACAGTTCCTCATTTGGTC TAGTTTTCGGGCCAGGGGAGTAAGTGAAATTCACT TCTCTATAAGAATAAGCCCATCCATCTCTTTTATTC ATGAACAGAGACAGAAAGAGTGCTTGGCATGGCA TGCCTCCGAGGGAGGCTGTGGGAGGAGGCCAAGCT GGCAGGCTCGGGTTCTTTGCCCTTGCTAGCGCCCA GGTCAGAGGGAAAAGGAATGTACTCGTACCCTTCC CTCACCACCTTGTAGGTCCCCAGGTACCAGGTGCC CAGGGAAGAAGGCCTTCACAATGATCCCCCCAGCT CAGAACAGCCCCTGCACACCCAGTAACCGGCATCT GGCTTCTCCTTAGCTTAGTGCAGCTGTGGATTCTGG GAAAGTGGCCTCTCTAATCTGAACTTG |
| SEQ ID NO: 33 | UTR3_8 1 | GCCAACCCAGACACAGTGCGGTACCAGAATTGCCT TAGAAGATACCAGAAGGTGCGGTCTAGGGACCAGT GAATAAGAAGACCCCACTTCAACTTCCCCTCGAAG ACGTTGTGCCTCACAGCGAGGCCTACAAGGGATGG GAACTTTTATTTCAAAATAAATACTACCGAGGCTG TGATCAGGCTCACCTTG |
| SEQ ID NO: 34 | UTR3_8 3 | AGGGGGTGGCAGGGCGGGTCATGGAGCTTTCGCCT GGGTGCTGTTGGTTGCTTGGTTGGCTGGTGGTTTGG GCAGGGGGAGTTAGGAGCTGGCAGGAGTGGGGCT GCATTTTTGAGGCCTTTGGGCATGATGGTGAAATG |
| | | TGTAGACTCGGGTACTCTGCCCAACCCCATGGGAA GTGTGTTGGCTTTCAACCAGCCTGCGTGTTTCTCCA CTGCTCAGCTCTTCTGGAGAGGTTGTCATCATTGTA TTTGTGGTTT |
| SEQ ID NO: 35 | UTR3_8 4 | GCATCCTGCTGCCTTCCCACAACATTAAACTCTCCT TCCTCAGTG |
| SEQ ID NO: 36 | UTR3_8 5 | GAGAACAGTCAGGCAGGGAGCCCTTCTCCCCACAG AGCTTTCAGGAGCAGACAGTGGCCTCCCCTTCCCC TCCCTGCCCCAGGACTTAAAGAGCAATAGTTTGCC CCTTTTGGAATGATCTCTGAATATATAAAACACAC ACACAAAGAGCAATA |

### Example 1: Preparation of RNA

1) Preparation of in vitro transcription template: A DNA sequence comprising T7 promoter, 5' UTR, target gene and 3' UTR was amplified using PCR for in vitro transcription. The specific constructs are T7 promoter (whose sequence is as set forth in SEQ ID NO: 37) + BamHI restriction enzyme cutting site (GGATCC) + 5UTR + KOZAK (GCCACC) + CDS + EcoRI restriction enzyme cutting site (GAATTC) + 3UTR + SpeI restriction enzyme cutting site (ACTAGT).
2) In vitro transcription: With PCR products as template and with rNTPs mix, DTT, T7 RNA polymerase, RNAase inhibitor, reaction buffer involved, the reaction system was allowed to react in a 37°C thermostat for 16h to complete in vitro transcription; DNase I was added into the reaction system, and the reaction system was allowed to react in a 37°C thermostat for 30min to digest the DNA template. The transcription product was purified by lithium chloride precipitation.
3) RNA capping: Uncapped RNA was predenatured at 65°C, then mixed with GTP, capping reaction liquid, S-adenosylmethionine, vaccinia capping enzyme, RNA enzyme inhibitor, 2'-o-methyltransferase at a certain ratio, and then allowed to react in a 37°C thermostat for a certain period of time to complete the capping of RNA. Purification was performed by lithium chloride precipitation.
4) RNA tailing: The capped RNA was subjected to tailing by using a polyA polymerase kit (Vazyme, DD4111) according to the instructions of the kit, and purified by lithium chloride precipitation after the reaction was completed.

### In vitro expression assay

1) Cell culture and in vitro transfection: The complete medium for HEK293T and Hela cells was DMEM high glucose medium (Hyclone) comprising 10% FBS (Hyclone), and the complete medium for A459 cells was RPMI 1640 medium (Gibco) comprising 10% FBS (Hyclne). One day before mRNA transfection, cells were seeded at 2*10⁴ cells/well in a 96-well cell culture plate (Corning). The next day, mRNA transfection was performed using Lipofectamine 3000 (Invitrogen). For the transfection method, refer to the instructions of Lipofectamine 3000.
2) GFP expression detection: After the cells were transfected for 16h, the 96-well plate was put into a microplate reader (Tecan), the system was excited at a wavelength of 488nm, and the OD value at a wavelength of 507nm was detected.
3) EPO (whose CDS sequence is as set forth in SEQ ID NO: 38) expression detection: After the cells were transfected for 16h, the supernatant was collected and ELISA was performed with the Quantikine^{®} IVD^{®} Human Erythropoietin ELISA (R&D) kit. For specific operations, refer to the instructions of the kit.

### Example 2: Expression level of GFP of different 5' UTRs in 293T cell lines (microplate reader):

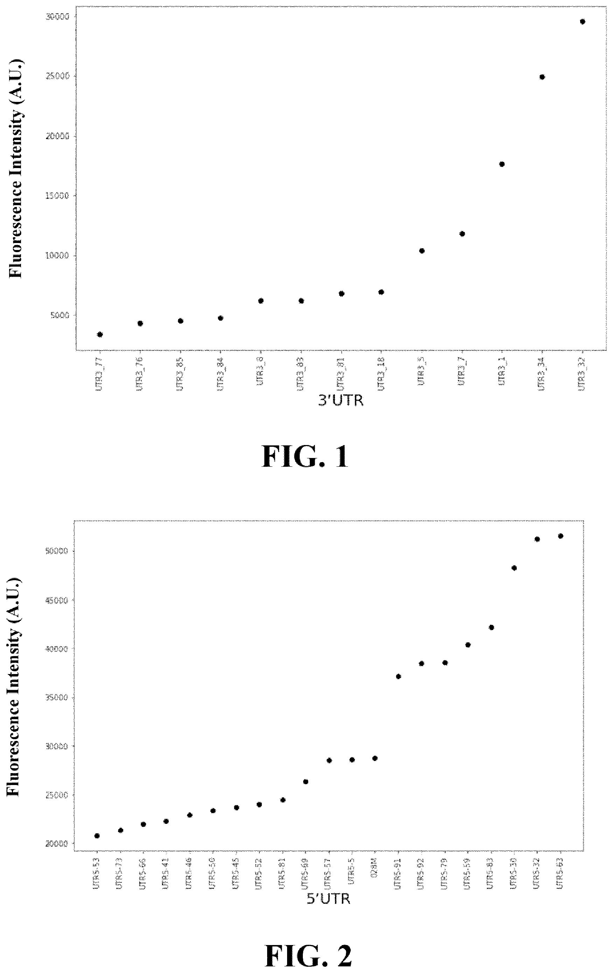

In order to test the effect of different 5' UTRs on the expression level of GFP (whose CDS sequence is as set forth in SEQ ID NO: 39), the 3' UTR was fixed as UTR3-1 in this example. The experimental results are shown in Table 2 below and FIG. 1.

**Table 2. Effect of different 5' UTRs on GFP expression level**

| Sample | Fluorescence intensity (A.U.) |
|---|---|
| UTR5-63 | 51555 |
| UTR5-32 | 51226 |
| UTR5-30 | 48250 |
| UTR5-83 | 42170 |
| UTR5-59 | 40445 |
| UTR5-79 | 38545 |
| UTR5-92 | 38456 |
| UTR5-91 | 37125 |
| 028M | 28735 |
| UTR5-5 | 28561 |
| UTR5-57 | 28478 |
| UTR5-69 | 26325 |
| UTR5-4 | 25863 |
| UTR5-81 | 24486 |
| UTR5-52 | 24031 |
| UTR5-45 | 23650 |
| UTR5-50 | 23388 |
| UTR5-46 | 22897 |
| UTR5-41 | 22258 |
| UTR5-66 | 21991 |
| UTR5-73 | 21332 |
| UTR5-53 | 20805 |

### Example 3: Expression level of GFP of different 3' UTRs in 293T cell lines (microplate reader):

In order to test the effect of different 3' UTRs on the expression level of GFP, the 5' UTR was fixed as 028M in this example. The experimental results are shown in Table 3 below and FIG. 2.

**Table 3. Effect of different 3' UTRs on GFP expression level**

| 3UTR | Fluorescence intensity (A.U.) |
|---|---|
| UTR3_32 | 29562 |
| UTR3_34 | 24980 |
| UTR3_1 | 17705 |
| UTR3_7 | 11850 |
| UTR3_5 | 10376 |
| UTR3_18 | 6982 |
| UTR3_81 | 6839 |
| UTR3_83 | 6218 |
| UTR3_8 | 6196 |
| UTR3_84 | 4743 |
| UTR3_85 | 4533 |
| UTR3_76 | 4323 |
| UTR3_77 | 3391 |
| UTR3_43 | 2790 |

### Example 4: Experiment on combinations of different 5' UTRs and different 3' UTRs

Sequences having good effects in Examples 2-3 (UTR5-32, UTR5-30, UTR5-83, UTR5-59, UTR5-92, UTR5-91, ABOC-028M; UTR3_32, UTR3_34, UTR3_1, UTR3_7, UTR3_5) were combined. After template preparation, in vitro transcription, RNA capping and tailing, and mRNA purity inspection, the preparation and detection of mRNA were completed. The qualified mRNA was transfected into HEK293T, Hela and A549 cells, respectively. The cell supernatant was removed 16h after transfection and ELISA was performed using the Quantikine^{®} IVD^{®} Human Erythropoietin ELISA (R&D) kit to test the expression level (in mIU/ml) of EPO (erythropoietin) in different cells. In total, 38 UTR and 3' UTR combinations of the present invention were tested in this example. The effects of various combinations of 5' UTR and 3' UTR on EPO expression level in HEK293T, Hela and A549 cell lines are shown in Tables 4, 5 and 6, respectively.

**Table 4. Effects of the various combinations on EPO expression level in HEK293T cell line**

| Combination No. | 5UTR | 3UTR | Expression level |
|---|---|---|---|
| Combination 1 | 028M | UTR3_1 | 105.6322712 |
| Combination 2 | 028M | UTR3_5 | 97.6679232 |
| Combination 3 | 028M | UTR3_7 | 78.4149776 |
| Combination 4 | 028M | UTR3_32 | 144.1282688 |
| Combination 5 | 028M | UTR3_34 | 123.816708 |
| Combination 6 | UTR5_30 | UTR3_1 | 128.8030824 |
| Combination 7 | UTR5_30 | UTR3_5 | 100.9427048 |
| Combination 8 | UTR5_30 | UTR3_32 | 126.4780864 |
| Combination 9 | UTR5_30 | UTR3_34 | 125.350216 |
| Combination 10 | UTR5_32 | UTR3_1 | 173.1066232 |
| Combination 11 | UTR5_32 | UTR3_5 | 119.8394808 |
| Combination 12 | UTR5_32 | UTR3_32 | 96.1739896 |
| Combination 13 | UTR5_32 | UTR3_34 | 123.6089424 |
| Combination 14 | UTR5_59 | UTR3_1 | 105.4937608 |
| Combination 15 | UTR5_59 | UTR3_5 | 91.9494224 |
| Combination 16 | UTR5_59 | UTR3_32 | 75.4172168 |
| Combination 17 | UTR5_59 | UTR3_34 | 80.9972072 |
| Combination 18 | UTR5_79 | UTR3_1 | 128.7239336 |
| Combination 19 | UTR5_79 | UTR3_5 | 105.0485488 |
| Combination 21 | UTR5_79 | UTR3_32 | 79.9089112 |
| Combination 22 | UTR5_79 | UTR3_34 | 73.260412 |
| Combination 23 | UTR5_83 | UTR3_1 | 90.326872 |
| Combination 24 | UTR5_83 | UTR3_5 | 91.563572 |
| Combination 25 | UTR5_83 | UTR3_7 | 97.944944 |
| Combination 26 | UTR5_83 | UTR3_32 | 98.8650488 |
| Combination 27 | UTR5_83 | UTR3_34 | 101.4472784 |
| Combination 28 | UTR5_91 | UTR3_1 | 105.3552504 |
| **Comparative Combination 1** | UTR5_91 | UTR3_5 | **74.0914744** |
| Combination 29 | UTR5_91 | UTR3_7 | 60.4086256 |
| Combination 30 | UTR5_91 | UTR3_32 | 125.548088 |
| Combination 31 | UTR5_91 | UTR3_34 | 94.3634608 |
| Combination 32 | UTR5_92 | UTR3_1 | 82.313056 |
| Combination 33 | UTR5_92 | UTR3_5 | 61.9124528 |
| **Comparative Combination 2** | UTR5_92 | UTR3_7 | **41.9174872** |
| Combination 34 | UTR5_92 | UTR3_32 | 77.118916 |
| Combination 35 | UTR5_92 | UTR3_34 | 49.169496 |

**Table 5. Effects of the various combinations on EPO expression level in Hela cell line**

| Combination No. | 5UTR | 3UTR | Expression level |
|---|---|---|---|
| Combination 1 | 028M | UTR3_1 | 85.7441635 |
| Combination 2 | 028M | UTR3_5 | 99.0018835 |
| Combination 3 | 028M | UTR3_7 | 67.380643 |
| Combination 4 | 028M | UTR3_32 | 134.805619 |
| Combination 5 | 028M | UTR3_34 | 120.3720655 |
| Combination 6 | UTR5_30 | UTR3_1 | 108.321745 |
| Combination 7 | UTR5_30 | UTR3_5 | 90.5421955 |
| Combination 8 | UTR5_30 | UTR3_32 | 111.2021425 |
| Combination 9 | UTR5_30 | UTR3_34 | 100.935301 |
| Combination 10 | UTR5_32 | UTR3_1 | 141.718573 |
| Combination 11 | UTR5_32 | UTR3_5 | 98.536285 |
| Combination 12 | UTR5_32 | UTR3_32 | 82.0982905 |
| Combination 13 | UTR5_32 | UTR3_34 | 105.970078 |
| Combination 14 | UTR5_59 | UTR3_1 | 64.950061 |
| Combination 15 | UTR5_59 | UTR3_5 | 92.412481 |
| Combination 16 | UTR5_59 | UTR3_32 | 69.0930985 |
| Combination 17 | UTR5_59 | UTR3_34 | 71.673619 |
| Combination 18 | UTR5_79 | UTR3_1 | 107.1616945 |
| Combination 19 | UTR5_79 | UTR3_5 | 102.5846245 |
| Combination 20 | UTR5_79 | UTR3_7 | 61.146358 |
| Combination 21 | UTR5_79 | UTR3_32 | 71.768317 |
| Combination 22 | UTR5_79 | UTR3_34 | 69.7086355 |
| Combination 23 | UTR5_83 | UTR3_1 | 82.421842 |
| Combination 24 | UTR5_83 | UTR3_5 | 86.193979 |
| Combination 25 | UTR5_83 | UTR3_7 | 85.688923 |
| Combination 26 | UTR5_83 | UTR3_32 | 86.7227095 |
| Combination 27 | UTR5_83 | UTR3_34 | 98.2127335 |
| Combination 28 | UTR5_91 | UTR3_1 | 80.5673395 |
| **Comparative Combination 1** | UTR5_91 | UTR3_5 | **57.784579** |
| Combination 29 | UTR5_91 | UTR3_7 | 48.172732 |
| Combination 30 | UTR5_91 | UTR3_32 | 120.2142355 |
| Combination 31 | UTR5_91 | UTR3_34 | 92.3888065 |
| Combination 32 | UTR5_92 | UTR3_1 | 80.7883015 |
| Combination 33 | UTR5_92 | UTR3_5 | 54.201838 |
| **Comparative Combination 2** | UTR5_92 | UTR3_7 | **43.9665625** |
| Combination 34 | UTR5_92 | UTR3_32 | 79.154761 |
| Combination 35 | UTR5_92 | UTR3_34 | 70.631941 |

**Table 6. Effects of the various combinations on EPO expression level in A549 cell line**

| Combination No. | 5UTR | 3UTR | Expression level |
|---|---|---|---|
| Combination 1 | 028M | UTR3_1 | 16.0613764 |
| Combination 2 | 028M | UTR3_5 | 22.6623136 |
| Combination 4 | 028M | UTR3_32 | 30.8543858 |
| Combination 5 | 028M | UTR3_34 | 20.862058 |
| Combination 6 | UTR5_30 | UTR3_1 | 30.090641 |
| Combination 7 | UTR5_30 | UTR3_5 | 24.635321 |
| Combination 8 | UTR5_30 | UTR3_32 | 27.9903428 |
| Combination 9 | UTR5_30 | UTR3_34 | 19.7619018 |
| Combination 10 | UTR5_32 | UTR3_1 | 23.4351506 |
| Combination 11 | UTR5_32 | UTR3_5 | 20.3801714 |
| Combination 12 | UTR5_32 | UTR3_32 | 16.9887808 |
| Combination 13 | UTR5_32 | UTR3_34 | 20.1892352 |
| Combination 14 | UTR5_59 | UTR3_1 | 16.4250644 |
| Combination 15 | UTR5_59 | UTR3_5 | 18.5344548 |
| Combination 16 | UTR5_59 | UTR3_32 | 17.5252206 |
| Combination 18 | UTR5_79 | UTR3_1 | 23.1987534 |
| Combination 19 | UTR5_79 | UTR3_5 | 19.8801004 |
| Combination 21 | UTR5_79 | UTR3_32 | 15.5703976 |
| Combination 22 | UTR5_79 | UTR3_34 | 14.1701988 |
| Combination 23 | UTR5_83 | UTR3_1 | 13.8246952 |
| Combination 24 | UTR5_83 | UTR3_5 | 12.815461 |
| Combination 25 | UTR5_83 | UTR3_7 | 10.3787514 |
| Combination 26 | UTR5_83 | UTR3_32 | 20.452909 |
| Combination 27 | UTR5_83 | UTR3_34 | 19.634611 |
| Combination 28 | UTR5_91 | UTR3_1 | 22.5622994 |
| **Comparative Combination 1** | UTR5_91 | UTR3_5 | **17.270639** |
| Combination 29 | UTR5_91 | UTR3_7 | 2.086665 |
| Combination 30 | UTR5_91 | UTR3_32 | 22.5622994 |
| Combination 31 | UTR5_91 | UTR3_34 | 15.7158728 |
| Combination 32 | UTR5_92 | UTR3_1 | 12.8881986 |
| Combination 33 | UTR5_92 | UTR3_5 | 10.3060138 |
| **Comparative Combination 2** | UTR5_92 | UTR3_7 | **10.0241556** |
| Combination 34 | UTR5_92 | UTR3_32 | 11.8698722 |
| Combination 35 | UTR5 92 | UTR3_34 | 6.950992 |

The aforementioned examples demonstrate that compared to **Comparative Combination 1,** in 293T cells, the combinations 1-19, 21, 23-28, 30-32 and 34 of the present invention have significantly higher EPO expression, up to about 2.3 times that of Comparative Combination 1 (the combination 10 of the present invention); in Hela cells, the combinations 1-28, 30-32 and 34 of the present invention have significantly higher EPO expression, up to about 2.8 times that of Comparative Combination 1 (the combination 10 of the present invention); and in A549 cells, the combinations 2, 4-11, 13, 15, 16, 18, 19, 23, 26-28, 30 of the present invention have significantly higher EPO expression, up to about 1.8 times that of Comparative Combination 1 (the combination 4 of the present invention).

Based on these data, it can be concluded that when the 5' UTR is selected from an RNA sequence corresponding to the nucleic acid sequence set forth in SEQ ID NO: 19 or 22 and the 3' UTR is selected from an RNA sequence corresponding to the nucleic acid sequence set forth in SEQ ID NO: 23, 24, 25, 28 or 29; when the 5' UTR is selected from an RNA sequence corresponding to the nucleic acid sequence set forth in SEQ ID NO: 3, 4 or 12, and the 3' UTR is selected from an RNA sequence corresponding to the nucleic acid sequence set forth in SEQ ID NO: 23, 24, 28 or 29; when the 5' UTR is an RNA sequence corresponding to the nucleic acid sequence set forth in SEQ ID NO: 17, and the 3' UTR is selected from an RNA sequence corresponding to the nucleic acid sequence set forth in SEQ ID NO: 23, 24 or 28; or when the 5' UTR is selected from an RNA sequence corresponding to the nucleic acid sequence set forth in SEQ ID NO: 20 or 21, and the 3' UTR is selected from an RNA sequence corresponding to the nucleic acid sequence set forth in SEQ ID NO: 23, 28 or 28, the 5' UTR and 3' UTR combination can significantly improve the translation efficiency of the coding sequence of the target gene, thereby significantly increasing the expression level thereof.

The aforementioned examples further demonstrate that compared to **Comparative Combination 2,** in 293T cells, the 38 UTR and 3' UTR combinations of the present invention have significantly higher EPO expression, up to about 4.1 times that of Comparative Combination 2 (the combination 10 of the present invention); in Hela cells, the combinations 1-33, 34 and 35 of the present invention have significantly higher EPO expression, up to about 3.2 times that of Comparative Combination 2 (the combination 10 of the present invention); and in A549 cells, the combinations 1, 2, 4-19, 21-28, 30-35 of the present invention have significantly higher EPO expression, up to about 3.1 times that of Comparative Combination 1 (the combination 4 of the present invention).

Based on these data, it can be concluded that when the 5' UTR is selected from an RNA sequence corresponding to the nucleic acid sequence set forth in SEQ ID NO: 3, 19 or 22 and the 3' UTR is selected from an RNA sequence corresponding to the nucleic acid sequence set forth in SEQ ID NO: 23, 24, 25, 28 or 29; when the 5' UTR is selected from an RNA sequence corresponding to the nucleic acid sequence set forth in SEQ ID NO: 4, 12, 17, 20 or 21, and the 3' UTR is selected from an RNA sequence corresponding to the nucleic acid sequence set forth in SEQ ID NO: 23, 24, 28 or 29; or when the 5' UTR is an RNA sequence corresponding to the nucleic acid sequence set forth in SEQ ID NO: 19, and the 3' UTR is selected from an RNA sequence corresponding to the nucleic acid sequence set forth in SEQ ID NO: 24, 25, 28 or 29, the 5' UTR and 3' UTR combination can significantly improve the translation efficiency of the coding sequence of the target gene, thereby significantly increasing the expression level thereof.

### Example 5: In vivo assay

Some combinations in Example 4 (as shown in Table 7) were tested in vivo. The CDS sequence thereof is as set forth in SEQ ID NO: 38. The preparation and detection of the mRNA were completed after template preparation, in vitro transcription, RNA capping and tailing, and mRNA purity inspection to obtain samples 1-8.

### Preparation and detection of lipid nanoparticles

Cationic lipid, DSPC, cholesterol and PEG-lipid were dissolved in ethanol at a molar ratio of 50:10:38.5:1.5, and mRNA was diluted in a citrate buffer (pH=4) at 10-50 mM. Liposomes were prepared by mixing an ethanol lipid solution and an aqueous solution of mRNA at a volume ratio of 1: 3 using a micro-fluidic splitter. The total dialysis flow rate was 9-30mL/min, thereby removing ethanol and replacing it with DPBS. Finally, the lipid nanoparticles were filtered through a 0.2 µm sterile filter.

### Animal study

Lipid nanoparticles comprising encapsulated samples 1-8 of human erythropoietin (hEPO) mRNA were systematically administered to female ICR mice aged 6-8 weeks by tail vein injection (Xipuer-Bikai, Shanghai) at a dose of 0.5mg/kg (wherein sample 8 is Comparative Combination 2 described in Example 4 as a control). Mouse blood was collected 6 hours after administration, and the blood sample was rapidly frozen and stored at -80°C for analysis. ELSA analysis was performed using a commercially available kit (DEP00, R&D system) according to the manufacturer's instructions.

The following table lists the characteristics of the tested lipid nanoparticles, including the hEPO expression levels measured from the test group (µg/ml).

**Table 7**

| No. | 5UTR | 3UTR | Expression level |
|---|---|---|---|
| Sample 1 | 028M | UTR3_1 | 1.66±0.12 |
| Sample 2 | 028M | UTR3_32 | 2.38±1.01 |
| Sample 3 | 028M | UTR3_34 | 2.07±0.14 |
| Sample 4 | UTR5_30 | UTR3_1 | 1.50±0.26 |
| Sample 5 | UTR5_30 | UTR3_32 | 1.69±0.08 |
| Sample 6 | UTR5_32 | UTR3_1 | 2.40±0.11 |
| Sample 7 | UTR5_32 | UTR3_32 | 2.10±0.27 |
| Sample 8 | UTR5_92 | UTR3_7 | 1.29±0.09 |

The results of the aforementioned in vivo assay show that, in accordance with the results of the in vivo assay, the 5' UTR and 3' UTR combination can also significantly improve the translation efficiency of the coding sequence of the target gene in vivo, thereby significantly improving the expression level thereof.

Although various embodiments of the present invention have been described above, it should be understood that they are provided in an exemplary manner only, not as a limitation. Without departing from the spirit and scope of the present invention, the present invention may be subject to various changes and modifications, which will fall within the scope of the present invention as claimed. The scope of protection claimed by the present invention is defined by the appended claims and their equivalents.

## Claims

1. An mRNA molecule comprising 5' UTR and 3' UTR, wherein the 5' UTR is selected from the following (1)-(5):
(1) 5' UTR comprising an RNA sequence corresponding to any of the nucleic acid sequences set forth in SEQ ID NOs: 1-22, a homologue, fragment or variant thereof, wherein the homologue, fragment or variant has the same or better function of improving translation efficiency compared with 5' UTR comprising an RNA sequence corresponding to any of the nucleic acid sequences set forth in SEQ ID NOs: 1-22; preferably, the nucleic acid sequence of the homologue has at least 70%, 80%, 85%, 90%, 91%, 92%, 93%, 94%, 95%, 96%, 97%, 98% or 99% identity with an RNA sequence corresponding to any of the nucleic acid sequences set forth in SEQ ID NOs: 1-22;
(2) 5' UTR consisting of an RNA sequence corresponding to any of the nucleic acid sequences set forth in SEQ ID NOs: 1-22;
(3) 5' UTR consisting of an RNA sequence corresponding to any of the nucleic acid sequences set forth in SEQ ID NOs: 4, 3, 12, 17 or 19-22;
(4) 5' UTR obtained from two or more identical 5' UTRs in the aforementioned (1)-(3) linked in tandem; or
(5) 5' UTR obtained from two or more different 5' UTRs in the aforementioned (1)-(3) linked in tandem,
and wherein the 3' UTR is selected from the following (1)-(5):
(1) 3' UTR comprising an RNA sequence corresponding to any of the nucleic acid sequences set forth in SEQ ID NOs: 23-36, a homologue, fragment or variant thereof, wherein the homologue, fragment or variant has the same or better function of improving translation efficiency compared with 3' UTR comprising an RNA sequence corresponding to any of the nucleic acid sequences set forth in SEQ ID NOs: 23-36; preferably, the nucleic acid sequence of the homologue has at least 70%, 80%, 85%, 90%, 91%, 92%, 93%, 94%, 95%, 96%, 97%, 98% or 99% identity with an RNA sequence corresponding to any of the nucleic acid sequences set forth in SEQ ID NOs: 23-36;
(2) 3' UTR consisting of an RNA sequence corresponding to any of the nucleic acid sequences set forth in SEQ ID NOs: 23-36;
(3) 3' UTR consisting of an RNA sequence corresponding to any of the nucleic acid sequences set forth in SEQ ID NOs: 23, 24, 28 or 29;
(4) 3' UTR obtained from two or more identical 3' UTRs in the aforementioned (1)-(3) linked in tandem; or
(5) 3' UTR obtained from two or more different 3' UTRs in the aforementioned (1)-(3) linked in tandem;
and wherein the 5' UTR and 3' UTR combination does not include RNA sequences corresponding to the nucleic acid sequences set forth below: SEQ ID NO: 22 and SEQ ID NO: 23, SEQ ID NO: 20 and SEQ ID NO: 24, and SEQ ID NO: 21 and SEQ ID NO: 25.

2. The mRNA molecule according to claim 1, wherein the 5' UTR is selected from an RNA sequence corresponding to the nucleic acid sequence set forth in SEQ ID NO: 4, 3, 12, 17, 19, 20, 21 or 22.

3. The mRNA molecule according to claim 1, wherein the 3' UTR is selected from an RNA sequence corresponding to the nucleic acid sequence set forth in SEQ ID NO: 23, 24, 25, 28 or 29.

4. The mRNA molecule according to any one of claims 1-3, wherein
(1) the 5' UTR is selected from an RNA sequence corresponding to the nucleic acid sequence set forth in SEQ ID NO: 4, 3 or 12, and the 3' UTR is selected from an RNA sequence corresponding to the nucleic acid sequence set forth in SEQ ID NO: 23, 24, 28 or 29;
(2) the 5' UTR is selected from an RNA sequence corresponding to the nucleic acid sequence set forth in SEQ ID NO: 19 or 22, and the 3' UTR is selected from an RNA sequence corresponding to the nucleic acid sequence set forth in SEQ ID NO: 23, 24, 25, 28 or 29;
(3) the 5' UTR is an RNA sequence corresponding to the nucleic acid sequence set forth in SEQ ID NO: 17, and the 3' UTR is selected from an RNA sequence corresponding to the nucleic acid sequence set forth in SEQ ID NO: 23, 24 or 28;
(4) the 5' UTR is selected from an RNA sequence corresponding to the nucleic acid sequence set forth in SEQ ID NO: 20 or 21, and the 3' UTR is selected from an RNA sequence corresponding to the nucleic acid sequence set forth in SEQ ID NO: 23, 28 or 28;
(5) the 5' UTR is selected from an RNA sequence corresponding to the nucleic acid sequence set forth in SEQ ID NO: 3, 19 or 22, and the 3' UTR is selected from an RNA sequence corresponding to the nucleic acid sequence set forth in SEQ ID NO: 23, 24, 25, 28 or 29;
(6) the 5' UTR is selected from an RNA sequence corresponding to the nucleic acid sequence set forth in SEQ ID NO: 4, 12, 17, 20 or 21, and the 3' UTR is selected from an RNA sequence corresponding to the nucleic acid sequence set forth in SEQ ID NO: 23, 24, 28 or 29; or
(7) the 5' UTR is an RNA sequence corresponding to the nucleic acid sequence set forth in SEQ ID NO: 19, and the 3' UTR is selected from an RNA sequence corresponding to the nucleic acid sequence set forth in SEQ ID NO: 24, 25, 28 or 29.

5. The mRNA molecule according to any one of claims 1-3, wherein
(1) the 5' UTR is an RNA sequence corresponding to the nucleic acid sequence set forth in SEQ ID NO: 4, and the 3' UTR is an RNA sequence corresponding to the nucleic acid sequence set forth in SEQ ID NO: 23;
(2) the 5' UTR is an RNA sequence corresponding to the nucleic acid sequence set forth in SEQ ID NO: 3, and the 3' UTR is an RNA sequence corresponding to the nucleic acid sequence set forth in SEQ ID NO: 23;
(3) the 5' UTR is an RNA sequence corresponding to the nucleic acid sequence set forth in SEQ ID NO: 3, and the 3' UTR is an RNA sequence corresponding to the nucleic acid sequence set forth in SEQ ID NO: 24;
(4) the 5' UTR is an RNA sequence corresponding to the nucleic acid sequence set forth in SEQ ID NO: 3, and the 3' UTR is an RNA sequence corresponding to the nucleic acid sequence set forth in SEQ ID NO: 25;
(5) the 5' UTR is an RNA sequence corresponding to the nucleic acid sequence set forth in SEQ ID NO: 3, and the 3' UTR is an RNA sequence corresponding to the nucleic acid sequence set forth in SEQ ID NO: 28;
(6) the 5' UTR is an RNA sequence corresponding to the nucleic acid sequence set forth in SEQ ID NO: 3, and the 3' UTR is an RNA sequence corresponding to the nucleic acid sequence set forth in SEQ ID NO: 29;
(7) the 5' UTR is an RNA sequence corresponding to the nucleic acid sequence set forth in SEQ ID NO: 4, and the 3' UTR is an RNA sequence corresponding to the nucleic acid sequence set forth in SEQ ID NO: 24;
(8) the 5' UTR is an RNA sequence corresponding to the nucleic acid sequence set forth in SEQ ID NO: 4, and the 3' UTR is an RNA sequence corresponding to the nucleic acid sequence set forth in SEQ ID NO: 28;
(9) the 5' UTR is an RNA sequence corresponding to the nucleic acid sequence set forth in SEQ ID NO: 4, and the 3' UTR is an RNA sequence corresponding to the nucleic acid sequence set forth in SEQ ID NO: 29;
(10) the 5' UTR is an RNA sequence corresponding to the nucleic acid sequence set forth in SEQ ID NO: 12, and the 3' UTR is an RNA sequence corresponding to the nucleic acid sequence set forth in SEQ ID NO: 23;
(11) the 5' UTR is an RNA sequence corresponding to the nucleic acid sequence set forth in SEQ ID NO: 12, and the 3' UTR is an RNA sequence corresponding to the nucleic acid sequence set forth in SEQ ID NO: 24;
(12) the 5' UTR is an RNA sequence corresponding to the nucleic acid sequence set forth in SEQ ID NO: 12, and the 3' UTR is an RNA sequence corresponding to the nucleic acid sequence set forth in SEQ ID NO: 28;
(13) the 5' UTR is an RNA sequence corresponding to the nucleic acid sequence set forth in SEQ ID NO: 12, and the 3' UTR is an RNA sequence corresponding to the nucleic acid sequence set forth in SEQ ID NO: 29;
(14) the 5' UTR is an RNA sequence corresponding to the nucleic acid sequence set forth in SEQ ID NO: 17, and the 3' UTR is an RNA sequence corresponding to the nucleic acid sequence set forth in SEQ ID NO: 23;
(15) the 5' UTR is an RNA sequence corresponding to the nucleic acid sequence set forth in SEQ ID NO: 17, and the 3' UTR is an RNA sequence corresponding to the nucleic acid sequence set forth in SEQ ID NO: 24;
(16) the 5' UTR is an RNA sequence corresponding to the nucleic acid sequence set forth in SEQ ID NO: 17, and the 3' UTR is an RNA sequence corresponding to the nucleic acid sequence set forth in SEQ ID NO: 25;
(17) the 5' UTR is an RNA sequence corresponding to the nucleic acid sequence set forth in SEQ ID NO: 17, and the 3' UTR is an RNA sequence corresponding to the nucleic acid sequence set forth in SEQ ID NO: 28;
(18) the 5' UTR is an RNA sequence corresponding to the nucleic acid sequence set forth in SEQ ID NO: 17, and the 3' UTR is an RNA sequence corresponding to the nucleic acid sequence set forth in SEQ ID NO: 29;
(19) the 5' UTR is an RNA sequence corresponding to the nucleic acid sequence set forth in SEQ ID NO: 19, and the 3' UTR is an RNA sequence corresponding to the nucleic acid sequence set forth in SEQ ID NO: 23;
(20) the 5' UTR is an RNA sequence corresponding to the nucleic acid sequence set forth in SEQ ID NO: 19, and the 3' UTR is an RNA sequence corresponding to the nucleic acid sequence set forth in SEQ ID NO: 24;
(21) the 5' UTR is an RNA sequence corresponding to the nucleic acid sequence set forth in SEQ ID NO: 19, and the 3' UTR is an RNA sequence corresponding to the nucleic acid sequence set forth in SEQ ID NO: 25;
(22) the 5' UTR is an RNA sequence corresponding to the nucleic acid sequence set forth in SEQ ID NO: 19, and the 3' UTR is an RNA sequence corresponding to the nucleic acid sequence set forth in SEQ ID NO: 28;
(23) the 5' UTR is an RNA sequence corresponding to the nucleic acid sequence set forth in SEQ ID NO: 19, and the 3' UTR is an RNA sequence corresponding to the nucleic acid sequence set forth in SEQ ID NO: 29;
(24) the 5' UTR is an RNA sequence corresponding to the nucleic acid sequence set forth in SEQ ID NO: 20, and the 3' UTR is an RNA sequence corresponding to the nucleic acid sequence set forth in SEQ ID NO: 23;
(25) the 5' UTR is an RNA sequence corresponding to the nucleic acid sequence set forth in SEQ ID NO: 20, and the 3' UTR is an RNA sequence corresponding to the nucleic acid sequence set forth in SEQ ID NO: 28;
(26) the 5' UTR is an RNA sequence corresponding to the nucleic acid sequence set forth in SEQ ID NO: 20, and the 3' UTR is an RNA sequence corresponding to the nucleic acid sequence set forth in SEQ ID NO: 29;
(27) the 5' UTR is an RNA sequence corresponding to the nucleic acid sequence set forth in SEQ ID NO: 21, and the 3' UTR is an RNA sequence corresponding to the nucleic acid sequence set forth in SEQ ID NO: 23;
(28) the 5' UTR is an RNA sequence corresponding to the nucleic acid sequence set forth in SEQ ID NO: 21, and the 3' UTR is an RNA sequence corresponding to the nucleic acid sequence set forth in SEQ ID NO: 24;
(29) the 5' UTR is an RNA sequence corresponding to the nucleic acid sequence set forth in SEQ ID NO: 21, and the 3' UTR is an RNA sequence corresponding to the nucleic acid sequence set forth in SEQ ID NO: 28;
(30) the 5' UTR is an RNA sequence corresponding to the nucleic acid sequence set forth in SEQ ID NO: 22, and the 3' UTR is an RNA sequence corresponding to the nucleic acid sequence set forth in SEQ ID NO: 24;
(31) the 5' UTR is an RNA sequence corresponding to the nucleic acid sequence set forth in SEQ ID NO: 22, and the 3' UTR is an RNA sequence corresponding to the nucleic acid sequence set forth in SEQ ID NO: 25;
(32) the 5' UTR is an RNA sequence corresponding to the nucleic acid sequence set forth in SEQ ID NO: 22, and the 3' UTR is an RNA sequence corresponding to the nucleic acid sequence set forth in SEQ ID NO: 28; or
(33) the 5' UTR is an RNA sequence corresponding to the nucleic acid sequence set forth in SEQ ID NO: 22, and the 3' UTR is an RNA sequence corresponding to the nucleic acid sequence set forth in SEQ ID NO: 29.

6. The mRNA molecule according to any one of claims 1-3, wherein the 5' UTR is an RNA sequence corresponding to the nucleic acid sequence set forth in SEQ ID NO: 4, and the 3' UTR is an RNA sequence corresponding to the nucleic acid sequence set forth in SEQ ID NO: 23.

7. The mRNA molecule according to any one of claims 1-3, wherein the 5' UTR is an RNA sequence corresponding to the nucleic acid sequence set forth in SEQ ID NO: 22, and the 3' UTR is an RNA sequence corresponding to the nucleic acid sequence set forth in SEQ ID NO: 28.

8. The mRNA molecule according to any one of claims 1-3, further comprising a nucleic acid sequence encoding a target polypeptide.

9. The mRNA molecule according to any one of claims 1-3, further comprising polyA.

10. Use of 5' UTR and 3' UTR in increasing the protein expression level or translation efficiency of mRNA, wherein the 5' UTR and 3' UTR are defined as in any one of claims 1-7.

11. A DNA encoding the mRNA molecule according to any one of claims 1-9.

12. A vector comprising the DNA according to claim 11.

13. A host cell comprising the vector according to claim 12.

14. A lipid nanoparticle comprising the mRNA molecule according to any one of claims 1-9.

15. A pharmaceutical composition comprising the mRNA molecule according to any one of claims 1-9, the DNA according to claim 11, the vector according to claim 12, the host cell according to claim 13 or the lipid nanoparticle according to claim 14, and a pharmaceutically acceptable carrier.

16. Use of the mRNA molecule according to any one of claims 1-9, the DNA according to claim 11, the vector according to claim 12, the host cell according to claim 13, the lipid nanoparticle according to claim 14 or the pharmaceutical composition according to claim 15 in the preparation of a drug for gene therapy, gene vaccination, protein replacement therapy, antisense therapy, or therapy by interfering RNA.
